# EUROPEAN PATENT SPECIFICATION

(11) **EP 2 575 496 B1**
(45) Date of publication and mention of the grant of the patent: **25.01.2017**
(21) Application number: 11725209.8
(22) Date of filing: 30.05.2011
(51) Int. Cl.: A23J 1/10, A23L 29/281, A61K 38/39, A61P 3/04, A23L 33/00, A23L 33/18, A61K 9/16, C07K 14/78

(54) **Collagen Powder**
Kollagenpulver
Poudre de collagène

(30) Priority: 28.05.2010 EP 10164379
(43) Date of publication of application: 10.04.2013
(73) Proprietor: HFP Ingrediënts B.V., 9728 JT Groningen (NL)
(72) Inventor: TEN KATE, Jan Gert, NL-9766 TW Eelderwolde (NL); VISSER, Petrus Rimke, NL-6524 DM Nijmegen (NL)
(74) Representative: V.O.
(86) International application number: PCT/NL2011/050381
(87) International publication number: WO 2011/149356

(56) References cited:
- EP-A1- 1 270 672
- WO-A1-2009/008714
- WO-A2-92/10944
- DE-U1-202010 009 512
- US-A- 5 162 506
- US-A- 5 411 887
- US-A- 5 484 888
- US-A1- 2005 267 292

## Description

The invention is directed to a method for preparing a collagen powder and to collagen powder that can be used for the preparation of a satiety inducing food product. The collagen powder of the invention is suitable as a food additive in food products for the reduction of hunger and as a possible treatment of obesity, which is the main risk factor for the development of metabolic syndrome.

Collagen is the major fibrous protein in animals. It is probably the most abundant animal protein in nature. Collagen has a molecular weight of about 300 000 Dalton, and is a combination of three polypeptide strands of the triple helix, wherein each strand is coiled into helical structure. Collagen comprises of a large number of fibres which, in turn, further comprise of a much greater number of fibrils of submicroscopic size. The fibrils have a diameter in the order of 1-5 nm and lengths ranging from several hundreds up to hundred thousands of nm.

If collagen is partially hydrolysed, the three polypeptide strands of the collagen molecules (tropocollagen strands) separate into globular, random coils, producing gelatine, which is used in many foods, including flavoured gelatine desserts. Besides food, gelatine has been used in pharmaceutical, cosmetic, and photography industries.

Due to the ever increasing prevalence of obesity world-wide and the associated diseases, much attention has been focused on strategies to combat the problem. Such strategies for weight management are directed to intensifying the metabolism, inhibit digestion and/or reducing calorie and food intake.

One type of food product that is applicable in the reduction of calorie intake are food additives that induce a satiety effect more quickly and reduce the appetite. This effect of satiety arises due to food intake and persists until the stomach is emptied. Due this effect, a person no longer has an appetite and is less likely to consume more food. Therefore food products that persist longer in the stomach and are slowly digested have a longer lasting satiety effect.

Recent interest has focused on collagen material, due its satiety inducing properties, as being a suitable additive in weight loss food products.

WO-A-2009/008714 describes that collagen hydrolysate can be used for the preparation of an edible composition for limiting voluntary food intake. The preferred average molecular weight of the collagen hydrolysate is described as being between 2 and 10 kDalton. The collagen hydrolysate is described as being obtained by controlled hydrolysis of gelatin which is obtained from animal collagen. Possible sources of animal collagen are not specifically described.

WO-A-2005/023017 describes a food composition comprising from 5 to 30 wt.% hydrolysed gelatin and a tryptophan source which is suitable to be used in meal replacement products. The preferred average molecular weight of the hydrolysed gelatin is disclosed as being from 2 to 10 kDalton. The hydrolyzed gelatin is said to be able to be prepared by enzymatic (proteolytic) treatment and to have different properties on its source and method of production.

EP-A-1 471 802 describes a satiety inducing food product, consisting of a cross-linked protein powder, that is subject to a reduced digestion speed in the stomach. The cross-linked powder is described as being obtained by crosslinking a protein into a gel, wherein said gel is dried. The preferred protein is described as being gelatin.

Collagen can be recovered from animal hide or skin, in particular from dehaired hide splits, such as bovine or from skins, such as porcine and poultry. A split refers to the inner or under layer of a hide or skin.

Several methods for the recovery of collagen from animal skin have been described in the prior art. EP-A-1 270 672 describes a process for recovering native collagen wherein hide or skin is comminuted in an aqueous suspension, liberated fat is separated, the pH of the suspension is made alkaline, a protease which does not degrade the native collagen is added, the suspension is acidified, the native collage is separated off and resuspended in water for disinfection and further purification, and finally the purified and disinfected native collagen is separated off as a solid phase. Collagen in the form of a powder is not disclosed.

US-A-3 932 677 describes a process for preparing an edible collagen from a limed hide collagen source, wherein hide collaged source is delimed by treatment with a dilute, edible acid at a pH of about 4.0 to 5.5, washing water soluble calcium salts from the hide collagen with water, forming an extrudable slurry from the neutralised and washed hide collagen and extruding said slurry through an annular die to form a tubular edible collagen casing.

WO-A-92/10944 describes a method in which raw hide splits are washed in dilute acid and one or more times with water to neutralise and remove the alkali, such as sodium sulphide and lime which are conventionally used to remove hair and residual fat. Before or after washing the hide splits are cut into pieces with typical dimensions of about 5-10 mm by 3-5 mm. These pieces are thereafter dried in a two stage drying process, wherein the first stage involves drying in a first stream of heated gas maintained at a temperature of less than about 60 °C, and the second stage involves drying in a second heated gas stream at a temperature which is higher than said first stream, wherein the temperatures and treatment times during the drying are controlled to minimize discoloration and hydrolysis of the dried product. Finally, the dried collagen is milled to form a fibrous product. Disadvantages of this product include that the collagen is hard to wet, the collagen has a low Bloom gel strength, and the bulk density of the collagen is low.

Other processes for producing collagen products are for instance known from US-A-5 411 887, US-A-2005/267292 and US-A-5 484 888.

Object of the invention is to provide a process for preparing a dry collagen powder.

Another object of the invention is to provide a collagen powder having improved satiety inducing properties for use in the preparation of food products.

Another object of the invention is to provide a collagen powder having improved gel strength.

A further object of the invention is to provide a collagen powder which can suitably be used as an emulsifier.

Yet another object of the invention is to provide a collagen powder which can suitably replace so-called "functional ingredients" used in foodstuffs, such as soy protein, soy isolates, caseinate, fosfates, alginates or the like. The inventor surprisingly found that this object can be met by a collagen powder having a specific Bloom gel strength.

A further object of the invention is to provide a collagen powder, *viz*. a dried product, that has not degraded or has degraded substantially less than known collagen powders. In accordance with the invention the triple helix forming the collagen molecule is untangled resulting in individual tropocollagen molecules, which make up the powder of the invention. Such a collagen powder was found to have superior properties compared to known collagen powders, as expressed for instance by its high Bloom gel strength.

The invention is as defined by the appended claims.

It was found that the desired properties of the collagen powder can be obtained by first converting hides or skins to a wet fibrous mass at a moderate temperature of 50 °C or less, followed by drying the wet fibrous mass at a high temperature, which is realized by contacting the wet fibrous mass with a roller dryer having a surface temperature of preferably 150 °C or higher for a time period of 2 minutes or less.

The low temperature of the first, wet stage is kept low, in order to minimize the hydrolysis of the collagen proteins. Also no enzymatic additions are required in this stage. This makes the process of the present invention fundamentally different from known processes, such as EP-A-1 270 672 and US-A-5 411 887. Subsequently the fibrous mass is dried at a high temperature of 150 °C or more. Surprisingly during the drying no, or only limited hydrolysis of the collagen material occurs, so that the dried collagen material is still composed of relatively large molecules, which explains the superior quality of the product of the present invention.

Accordingly, in a first aspect the invention is directed to a process for producing collagen in the form of a powder, which process comprises first producing a wet collagen product (fibrous mass) from a hide or skin at a temperature of 50 °C or less, followed by drying said wet collagen product at a roller dryer, which typically has a surface temperature of 150 °C or higher, for a time period of 2 minutes or less, to obtain said collagen powder. The wet collagen product is obtained typically by subjecting the hide or skin to process steps such as a size reduction step; an alkaline and/or an oxidizing treatment step; and a neutralizing step following said alkaline and/or an oxidizing treatment.

In a further aspect, the present invention is directed to a collagen powder, having a Bloom gel strength of 300 g or more as determined by standard test methods, in particular by ISO 9665. The "Bloom gel strength" is a measure of the ability of a material to form a gel. The Bloom gel strength is the weight in grams required to depress the gel a distance of 4 mm with a piston having a cross-sectional area of 1 cm², the gel first having been cooled for a defined time under defined conditions. Thus, the higher the Bloom gel strength of a material is, the greater the ability of that material to form a gel. The gel for this measurement has a standard concentration of 6.67% and has been kept 17 hours at 10 °C prior to measurement.

It was found that such a collagen powder according to the invention has advantageous hygroscopic qualities.

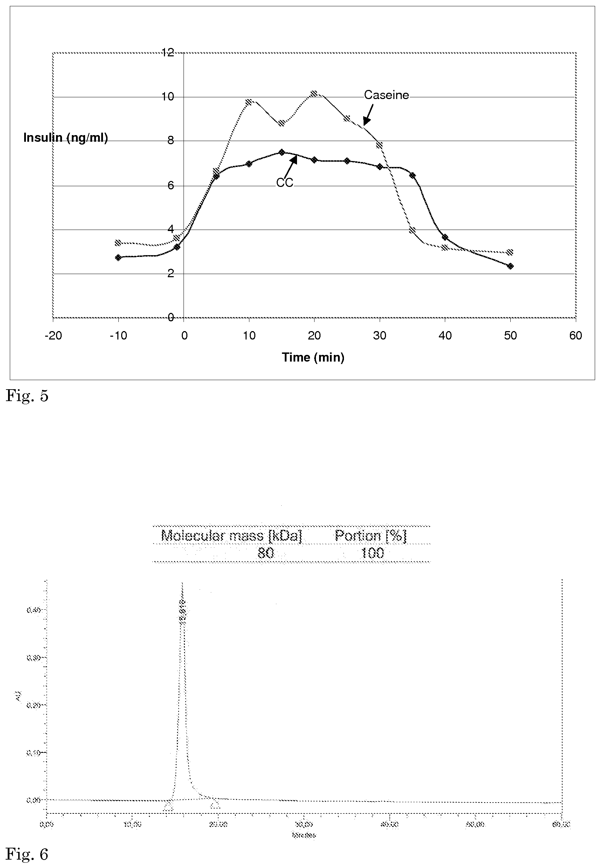

The collagen powder of the present invention is characterized by a very narrow distribution in molecular weight. It was found that 99 wt.% or more of the collagen particles has the same molecular mass. This may for instance be measured using gel permeation chromatography (GPC). Figure 6 shows the result of a typical GPC measurement on a collagen powder according to the present invention, in which a single peak is seen, indicative for a narrow distribution in molecular weight. Thus in accordance with the present invention the collagen molecules have a very narrow molecular weight distribution. Also the collagen molecules have a molecular weight that is considerably higher than collagen obtained according to prior art methods.

The collagen powder of the present invention has excellent heat stability, which makes it suitable for addition to food products that need to undergo subsequent heat treatment, e.g. pasteurization. Surprisingly the collagen powder of the present invention maintains its functionality with respect to emulsifying and water binding properties when subjected to such heat treatments.

Surprisingly, the collagen powder of the invention was found to have improved satiety inducing properties when used as a food supplement, compared to known products used for this purpose.

The excellent properties of the collagen powder of the invention are believed to be the result of the high nativity of the collagen. This means that the protein molecules making up the collagen powder are relatively large compared to collagen obtained by conventional processes. In a preferred embodiment, the collagen powder of the invention is of bovine origin and comprises particles having a molecular mass of about 80 kDa. Preferably more than 99 wt.% of the particles, more preferably about 100% of the particles have a molecular weight of about 80 kDa, as may be measured by using GPC, for instance resulting in the GPC pattern depicted in figure 6.

The collagen powder of the invention is suitable for the use as a food additive in food products for the reduction of hunger and weight management. It can thus be used as a possible treatment of obesity.

For good satiety inducing properties it is preferable that the food products comprises per dosage (*i.e.* unit to be consumed) at least 7 gram collagen powder of the invention and preferably less than 15 gram wt. %. Typically a food composition according to the present invention comprises 2 to 4 wt.% (based on dry weight) of the collagen of the invention.

Examples of suitable food products in which the collagen powder of the invention can be applied include but are not limited to the following: tablets, snacks, nutritional bars, ready made meals, baked products (*e*.*g*. muffins), meat products, instant soups, reconstitutable powder drinks, soups, drinks, yoghurts and health shakes.

The ingredients for such food products would be dependent on the type of food product. However typical ingredients can comprise such components as vegetable, fruit, eggs, meat or milk products, seeds, grains, pulses, fats or oils and water. The food product may also further comprise aromatic substances, colorings and flavorings, food salts, bulking agents, emulsifiers, saccharides, artificial and natural sweeteners such as sugars and sugar sources, carbohydrates, vitamins, alginates (*e*.*g*. sodium alginate) and minerals.

It was also found that such collagen powder has advantageous emulsifying properties. Furthermore, the collagen powder of the invention was found to be able to improve the structure of food products in which it is used. A relatively small addition, in the order of 1%, will significantly increase of the viscosity of meat emulsions. Substitution of phosphates in meat products by collagen powder without loss of thermal stability is another option.

Moreover, the collagen powder was found to be a suitable replacement of soy protein. This is advantageous in view of the growing demand for clean-label food products.

Collagen powder of the invention has a Bloom gel strength of at least 300 g, preferably at least 350 g, more preferably at least 400 g, and even more preferably at least 450 g. These Bloom gel strengths are superior than those mentioned in the prior art for other collagen products. The upper limit of the Bloom gel strength of the collagen powder of the invention can be high as 550 or even 600 g.

Preferably the collagen powder of the invention is a bovine collagen powder, in particular a powder comprising less than 1 wt.% fat (dry matter basis) and at least 95 wt.% protein (dry matter basis; as determined by N×5.52 method. For porcine and poultry collagen powder of the present invention the fat percentage is generally higher, *e.g.* 5-20 wt.%. Protein contents are typically 80 wt.% or more.

Unlike gelatin, the collagen powder of the present invention does not give a clear solution in water. It is preferred that the collagen powder of the invention has a powder structure, in particular a free flowing powder.

Preferably, the collagen of the invention has a high number average molecular weight, e.g. around 80 kDa for bovine collagen. For collagen of porcine or poultry origin, similar values of around 80 kDa are obtained.

The collagen powder of the present invention has remarkable emulsifying properties. For instance, using as little as 1 part of powder to 75 parts of water and 75 parts of oil or animal fats in a results in a stable warm emulsion (> 40 °C). Using vegetable oils a stable cold system (*viz.* less than 10 °C) can be obtained with as little as 1 part powder to 60 parts water and 60 vegetable oil.

The collagen powder of the invention gels already in a 1 : 15 ratio (wt./wt.) with cold water (< 10 °C). Gelling in cold water can be achieved in relative amounts of down to 5 wt.% collagen or even less. In hot water (> 40 °C) the collagen gel will may already form a firm gel in relative amounts as low as 3.5 wt.% or even down to 2.5 wt.%.

The collagen of the invention is remarkably heat stable. It will reform to a gel even after pasteurization/sterilization, which makes the protein very suitable for use in food products, in particular in canned, sterilized or pasteurized food products.

By virtue of its exceptionally high Bloom gel strength, the collagen powder of the invention can be advantageously used as a gelling agent. The collagen powder of the invention was found to keep a high bloom count (typically more than 225) even after heating to 100 °C.

In a further aspect the invention is directed to a method for preparing a collagen powder. This method is used to obtain the collagen powder described herein.

In the method of the invention, an animal hide or skin is subjected to a first stage size reduction, for example by using a standard meat grinder, bowl cutter, shredder or the like. This may be followed by a second stage size reduction in which the size of the skin or hide particles is further reduced. This step is followed by a step of dispersing the milled skin or hide in water, preferably in a stirred tank and then subjecting the milled skin or hide to an alkaline and/or oxidizing treatment. After this step, the pH of the dispersion is neutralized, *viz*. adjusted to about 5-7.

Next, the solids may be separated from the liquid, for example by centrifugation and/or using a screen. Then a third stage size reduction step may be carried out, which is followed by redispersing the fibrous mass in water. This may be followed by isolating the solids, for instance by centrifugation and/or screening. In accordance with the present invention this wet stage of the process the process is carried out at the low temperature of 50 °C or less, preferably below 45 °C, even more preferably at 42 ± 2 °C.

The wet stage is followed by the drying stage, which is carried out at the relatively high temperature. According to the invention this high temperature is obtained by means of a roller drying step, which assures relatively short contact times. In this high temperature drying step the surface temperature of the roller dryer is preferably kept at 150 °C or more, preferably 160 °C or more, more preferably 165 ± 4 °C. The drying may be followed milling and screening to obtain a powder product having the required particle size.

In one typical embodiment, the method of the invention comprises:
- providing animal hide or skin;
- size reduction in one or more steps;
- dispersing the particles in water;
- subjecting the dispersion to alkaline treatment and/or to oxidizing treatment, which results in the dispersion of hide or skin particles being converted into a fibrous mass;
- adjusting the pH of the fibrous mass to neutral (7) or slightly acidic pH;
- separating the solids and the liquid contained in said fibrous mass;
- subjecting the solids to a further size reduction step;
- dispersing the solids in water;
- again adjusting the pH to neutral or slightly acidic pH;
- again separating the solids and the liquid in said fibrous mass; and
- size reduction and/or particle separation to obtain a product having the desired particle size distribution.

Optionally a metal detection step is carried out before or during one of the first size reduction steps.

During the entire wet part of the method the temperature is maintained at a level so that the native collagen will not or only to a very small extent degrade, denature or gelatinize. The temperature depends to an extent on the type of collagen treated, but it should preferably not be greater than 50 °C. More preferably the temperature is not greater than 45 °C and even more preferably no greater than 40 °C. However, in one or more subsequent drying steps the temperature can be increased to much higher temperatures. One would expect that higher temperature would result in fragmentation of the collagen molecules. However the present inventors found that by using a roller dryer, contact time can be kept sufficiently short: in order of seconds to several minutes, *e*.*g*. from 10 seconds - 5 minutes, preferably 15 seconds to 2 minutes.

Thus the method of the present invention surprisingly allows the preparation of collagen powder having exceptionally high Bloom gel strength, as well as exceptional emulsifying and heat stability properties.

The applied animal hide or skin is preferably hide split or fresh skin. It can originate from various animals such as bovine, porcine and poultry and is preferably from bovine. Bovine hide splits that are limed are advantageous, because they have a relatively low fat content, typically less than 10 wt. % based on dry solids with a pH value from 10 to 12. Fresh porcine and poultry skins have a higher fat content, typically about 20-40 wt. %, based on the raw materials and have a high plate count.

The animal hide or skin is pre-ground in a first stage size reduction using a commercial grinder, such as a Wolfking™ or Meatwolf™ grinder. Preferably this results in ground hide or skin having an average size of several millimeters, for instance having a largest diameter of 2 to 20 mm, *e*.*g*. strips measuring 3-4 by 10-15 mm.

The ground animal hide or skin may then be subjected to metal detection, *e*.*g*. based on induction or magnetism. In a second stage size reduction, the animal hide or skin may be subjected to fine grinding typically to sizes of about 2-5 mm, *e.g.* 1-2 × 3-5 mm, using again for instance Wolfking™ or Meatwolf™ grinders.

The animal hide or skin is subjected to an alkaline treatment and/or an oxidizing treatment. The alkaline treatment is applied in order to remove residual fat. Usually, the alkaline treatment involves the application of an aqueous solution comprising an alkaline agent such as sodium hydroxide or calcium hydroxide. Preferably is the alkaline agent calcium hydroxide. Typically the concentration of calcium hydroxide used is from 1 to 2 % and is preferably 2 %. The pH during the alkaline treatment can be at least 10, preferably at least 11, more preferably at least 12. Preferably the pH during the alkaline treatment is no greater than 12.5. Since the bovine hide splits are already limed, only the fresh porcine and poultry skins are treated. The alkaline treatment can be performed very fast, *e*.*g*. within several seconds to minutes, e.g. typically less than 10-20 seconds.

The oxidizing treatment is applied in order to decontaminate the animal hide or skin, particularly the fresh porcine and poultry skins. In limed bovine hide splits, the treatment also oxidizes residual sulphide from the tanning process to suphite/sulphate. This prevents the production of hydrogen sulphide upon pH reduction in the neutralizing treatment. The oxidizing treatment is an aqueous solution comprising an oxidizing agent such as hydrogen peroxide or ozone. Preferably is the oxidizing agent hydrogen peroxide. Typically the hydrogen peroxide concentration is about 300 to 2500 ppm. The oxidizing treatment can be performed for several seconds to minutes, typically less than 10 seconds. The oxidizing treatment is preferably applied together with the alkaline treatment.

Following this, the resulting product is subjected to a pH adjustment to bring the pH at a neutral or slightly acidic pH 5-7. This can be done by acidifying the dispersion. This can involve washing the skin or hide in an aqueous solution of an acid. The purpose of neutralizing is, among others, the deliming of the skin or hide. During the neutralizing treatment water soluble calcium salts are removed from the hide. Preferred acids for neutralizing the hide or skin include lactic acid, hydrochloric acid, carbon dioxide, acetic acid, ethylene diamine tetraacetic acid, ammonium chloride, propionic acid, and fumaric acid. More preferably is carbon dioxide or acetic acid the preferred acid.

The pH should preferably not be lower than 4.2. It was found that optimal results are obtained if pH is kept between 5-6. The neutralizing treatment can be carried out very quick and can be completed in *e.g.* several seconds to about one minute.

The animal hide or skin is then subjected to a step for separating slits from the liquid, *e*.*g*. decanted or tri-canted. Following this, the animal hide or skin is again decanted or tri-canted to separate out the solids. The bovine collagen dry matter of the solids is typically between 10 to 30 %. The porcine and poultry collagen dry matter is typically 10 to 35 % and further comprises 2 to 7 % fat.

The neutralized animal skin or hide may then be subjected to a third stage size reduction where it is finely ground. As an optional step, the wet, ground porcine and poultry skin is isolated as a final product. It is a microbiologically stable product, comprising of a protein and fat content of about 20 and about 4 wt.% respectively.

Thereafter the ground product must be dried. Drying is carried out using a roller dryer. Other contact dryers are belt dryers or plate dryers. Using a drying roller, as well as drying the animal hide or skin after grounding, is advantageous in view of moisture transport. Because these measures provide excellent moisture transport, it suffices in general to dry the ground animal hide or skin on the roller dryer for a time period of 2 minutes or less, or even 1 minute or less, *e.g.* 10 to 60 seconds.

Because the drying is carried out at a relatively high temperature, this step can be performed quickly, which adds to process economy. In addition these short contact times are believed to add to maintaining the high molecular weight and narrow distribution in molecular weight of the product.

If the collagen is dried by means spin flash drying or air drying, this does not result in a dried product having the desirable properties.

As a final step, the collagen product is milled or sieved (screened). The milled and/or screened animal hide or skin can have an average particle size of 200 µm or less, preferably 100 µm or less. In a preferred embodiment, the average particle size of the ground animal hide or skin is in the range of 10-100 µm.

It was surprisingly found that storage of the ground animal hide or skin before drying has a detrimental effect to the Bloom gel strength of the resulting collagen powder. Without wishing to be bound by theory, the inventor believes that micro-organisms play a role in this degradation of the product. It is therefore preferred that the ground animal hide or skin is dried within 24 hours after grinding, preferably within 12 hours, more preferably within 6 hours, and most preferably directly after grinding.

Optionally, the dried collagen powder can be size separated, for instance by using one or more suitable sieves.

The method of the invention provides a collagen powder that has a substantially non-fibrous structure. In addition, the collagen powder has a relatively high bulk density of at least 540 g/l, preferably at least 550 g/l.

The invention is now elucidated on the basis of some examples, which are not intended to limit the scope of the invention.

### Examples

### Viscosity experiments

A 10 % gelatin solution was made according to EP-A-1 471 802. The gel solution was cross linked using transglutaminase (Active EB from Ajinomoto) at pH 6 for 24 hr at 40 °C. A firm mass formed that remained firm at a temperature at 40 °C. This in contrast to an untreated 10 % gelatin solution which is liquid at 40 °C.

A 5 g piece from the cross linked gelatin was transferred to a 100 ml solution further comprising one Betaine/Pepsine pill (from AOV Orthomolecular Food Supplement BV). The pH and temperature were 2.5 and 37 °C, respectively and were maintained for the duration of the experiment. After four hours, there was no detectable degradation of the gelatin. However this is not a realistic simulation of what happens in one stomach, crosslinked gelatin would be dry ground and possibly added to a food as a supplement. It would not be consumed as a big chunk.

100 g of crosslinked gelatin was resuspended in 100 ml water containing a Betaine/Pepsine pill in a blender at a pH of 2.5 and temperature of 37 °C. The viscosity of the suspension was measured with a Brookfield viscosimeter using a spindle 3 and a rotational speed or 50 rpm. At *t*= 0 min the viscosity was 1.2 Pa.s (1200cp) after 30 min the viscosity was < 0.04 Pa.s (40cp) and the gelatin was completely liquid. From this result we should not expect that the product of EP-A-1 471 802 to be very effective as a hunger suppressant.

A similar test was performed using Caseinate EM7 from DMV and the collagen powder of the invention, Kapro 95. In two separate solutions, each containing a Betaine/Pepsine pill, 20 g EM7 and 20 g Kapro 95 were suspended respectively. The solutions had a pH value of 2.5 and a temperature of 37 °C. Viscosity measurements were made with a Brookfield viscosimeter using a spindle 3 and a rotational speed or 50 rpm. The results are shown below (see table 1).

**Table 1.**

| (Please note that 100cp equals 1Pa.s) | | | | |
|---|---|---|---|---|
| Sample | Viscosity (*t*=0 min) | Viscosity (*t*=60 min) | Viscosity (*t*=120 min) | Viscosity (*t*=180 min) |
| EM7 | 1300 cp | 600 cp | 500 cp | 400 cp |
| Kapro 95 | 8300 cp | 6000 cp | 4000 cp | 4200 cp |

The results show that Kapro 95 remains highly viscous for a longer period time than EM7.

### Example 1. Short term effect of bovine collagen

The Example was performed using three groups of rats, comprising eight animals per group. One group received the standard rat diet (lab chow) and served as a general control. The experimental groups received a combination of 60 % lab chow and 40 % bovine collagen according to the invention (Collagen Care, abbreviated to CC). There was also a third group, who received a combination of 60 % lab chow supplemented with 40 % casein. This third group served as controls for the high protein diet of the experimental CC. During the experiments, the animals had food available for 20 hours a day.

Circadian Time, or CT, is a way to experimentally compare day and night activities of animals. In a 12 hours dark / light regime, CT 0-12 was defined as the inactive period (in humans is that the night, in rats is that the light period). CT 12-24, was conversely defined as the active period (in humans the light, in rats the dark). This was the experimental method used.

The animals were housed individually for three days and were food intake, water intake and changes in body weight measured. The exact experimental protocol was as follows:
- Circadian time CT 8: food is removed + body weight measured
- Food is returned on CT 12, food and water intake measured from CT 12 - CT 14 (meal two hours) and for CT 12 - CT 8 (food intake over 24 hours is determined.)

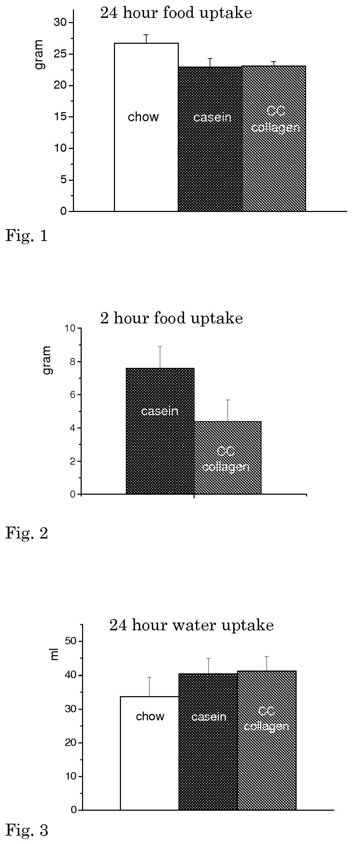

The provision of a high protein diet caused a reduction in food intake to about 85 % of that of the normal control diet (see figure 1). This effect is often seen in diets with a high protein content and is comparable with the effect observed of the popular Atkins diet from the USA. The satiating effect of the high protein diet resulted in the space of three days in a light but significant weight loss (5 %) compared to the control diet. This applied for both the CC diet and the casein diet.

Addition of bovine collagen led especially to a very sharp reduction in food intake in the first two hours (CT 12-14) after the food was returned (see figure 2). This was the most striking effect of the CC diet compared to the casein diet (and of course compared to the control diet).

Also the 24 hour water intake measurement was increased in the CC diet (see figure 3). The two hour water intake (CT 12-14) was not significantly different between the CC group and the control group. However if one looks at the so called prandial drinking (water intake per amount of food), then the CC animals drink relatively more.

### Example 2. Long term effect of bovine collagen

The Example was performed using four groups of rats, comprising eight animals per group. All animals received a standard lab chow diet enriched with either 20 % or 40 % protein. Two groups received bovine collagen according to the invention (CC), while the other two groups received casein. The four groups consisted of:
- CC 20: a combination of 80 % lab chow and 20 % bovine collagen
- CC 40: a combination of 60 % lab chow and 40 % bovine collagen
- 20 casein: a combination of 80 % lab chow and 20 % casein
- 40 casein: a combination of 60 % lab chow and 40 % casein
The casein groups served as controls for the high protein diet comprising of bovine collagen. During the experiments, the animals had food available ad lib.

The animals were housed individually and during a period of 14 days, measurements were taken twice daily at CT 9 was the 24-hour of food intake, water intake and changes in body weight.

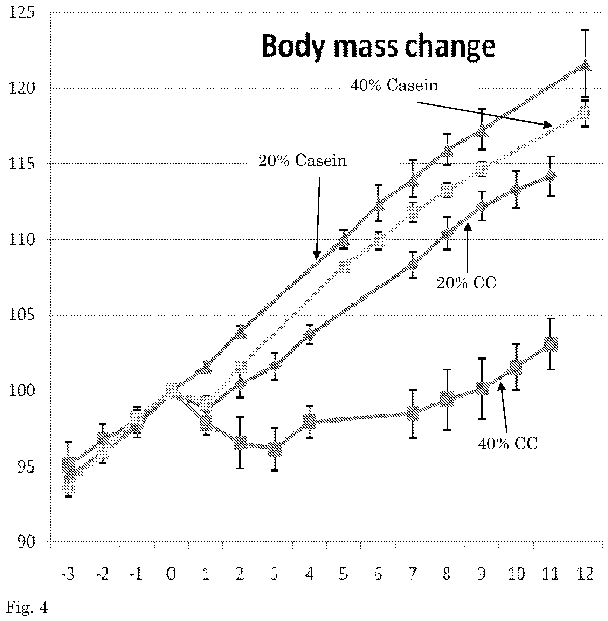

The results from the experiment were that the addition of bovine collagen reduced food intake and increased water intake. The data of the food and water intakes calculated cumulatively over 14 days were significantly different. Figure 4 shows the body weights over the first period. It is clear from figure 4 that the addition of bovine collagen results in a significant reduction in body weight over the longer term.

Both short and long term food intake in rats was greatly reduced by the consumption of diet enriched with bovine collagen according to the invention (CC). This is possible due to a combination of two properties of the CC diet, namely (a) the high protein content, and (b) a high Bloom number. Ultimately this leads to an increased stomach filling, inhibited gastric emptying and delays the absorption of food in the blood. This combination creates a highly reduced short term food intake leading to improved insulin profiles in the blood.

In the long term it appears that the bovine collagen according to the invention has an effect of food intake being reduced, leading to a significant reduction in body weight. The addition of bovine collagen in the normal diet has thus a reducing effect of food intake and body weight which means that this diet can potentially contribute to fighting and preventing obesity and its pathophysiological consequences, such as type 2 diabetes in humans and animals.

Finally, it should be noted that both the short and long term effects are very promising. The satiation effect is similar to that from an injection with a recognized satiety hormone like cholecystokinin. Addition of bovine collagen according to the invention to a regular diet appears to offer possibilities in the treatment of obesity and its pathophysiological consequences, such as metabolic syndrome.

### Example 3. Improved insulin profiles in the blood in an intravenous glucose tolerance test (IVGTT)

Rats were divided in two groups. The animals were housed individually during a period of 21 days. One group received a diet of 70% lab chow and 30% casein (*N*=4) and the other group (*N*=4) received a combination of 70% lab chow and 30% bovine collagen according to the invention (CC). During the IVGTT the rats were infused with 15 mg/min glucose over a 30 minutes period. Before the onset of the infusion, two baseline samples were taken at time points *t* = *-* 11 and - 1 min. After the start of the infusion at *t* = 0 min, blood samples were taken at time points t = 5, 10, 15, 20, 25, 30, 40 and 50 min.

Plasma insulin was analysed by radioimmunoassay.

Figure 5 shows the resulting insulin profiles of the blood in both groups during the IVGTT. In the CC fed rats, the invention caused a significant (*p* < 0.05) reduction in the areas under the curve (AUC) of insulin during the IVGTT.

It can be concluded that that the addition of bovine collagen to the normal diet results in improved insulin profiles in the blood which means that it may contribute to preventing type 2 diabetes in humans and animals.

## Claims

1. Method for preparing collagen powder, comprising the steps of:
producing at a temperature of 50 °C or less a wet collagen product by subjecting a hide or skin to a size reduction step; an alkaline and/or an oxidizing treatment step; and a neutralizing step; followed by drying of said wet collagen product for a time period of 2 minutes or less using a roller dryer to obtain said collagen powder.

2. Method according to claim 1, wherein said drying is carried out using a roller dryer having a surface temperature of more than 150 °C.

3. Method according to the previous claim, wherein the temperature of the steps for producing the wet collagen product is kept at 45 °C or less and preferably 40 °C or less.

4. Method according to any of the previous claims, wherein said drying of said wet collagen product is carried out at a surface temperature of said roller dryer of 155 °C or higher, preferably 160 °C or higher, more preferably 165 ± 4°C.

5. Method according to any of the previous claims, wherein said animal hide or skin is selected from the group consisting of bovine, porcine and poultry, preferably bovine.

6. Method according to any of the previous claims, wherein, said alkaline and/or oxidizing treatment is performed at a pH value of 10 or more, preferably 11 or more, more preferably 12 or more.

7. Method according to any of the previous claims, wherein the neutralizing treatment results in a pH value of 6 or less, preferably from 5 to 6.

8. Method according to any of the previous claims, wherein the ground animal hide or skin is dried within 24 hours after grinding, preferably within 12 hours, more preferably within 6 hours, and most preferably directly after grinding.

9. Collagen powder, which is a bovine, porcine or poultry collagen product, obtainable by a method according to any of the previous claims, having a Bloom gel strength of at least 300, as determined by standard test method ISO 9665 using a gel having a concentration of 6.67%, which has been kept for 17 hours at 10 °C prior to measurement.

10. Collagen powder according to the previous claim, which is a bovine collagen and which comprises less than 1 wt.% fat, dry matter basis, and at least 95 wt.% protein, dry matter basis; as determined by the *N*×5.52 method.

11. Collagen powder according to claim 9 or 10, wherein more than 99 wt.% collagen of the collagen molecules have the same molecular mass.

12. Collagen powder according to the previous claim, which is a bovine collagen powder product wherein the collagen has a molecular mass of about 80 kDa.

13. Collagen powder according to any of the claims 9-12, for use in the reduction of hunger and weight management, wherein preferably the food product is for the treatment of obesity and metabolic syndrome, in particular for treating insulin resistance.

14. Food product having a strong satiety effect, comprising up to 5 wt.% collagen powder according to any of the claims 9-12, preferably 2-4 wt. %, more preferably 3 ± 0.5 wt.% based on the dry weight.

15. Food product according to claim 14, wherein said food product comprises ingredients selected from vegetable, fruit, eggs, meat or milk products, seeds, grains, pulses, fats or oils, water, aromatic substances, colorings and flavorings, food salts, bulking agents, emulsifiers, saccharides, artificial and natural sweeteners such as sugars and sugar sources, carbohydrates, vitamins, minerals and combinations thereof.

## Patentansprüche

1. Verfahren zur Herstellung von Kollagenpulver, umfassend die Schritte von:
Erzeugung eines nassen Kollagenprodukts bei einer Temperatur von 50°C oder weniger, indem ein Fell oder eine Haut einem Reduktionsschritt; einem Alkali- und/ oder einem Oxidationsbehandlungsschritt; und einem Neutralisierungsschritt unterzogen wird; gefolgt von Trocknung des nassen Kollagenprodukts für eine Zeitdauer von 2 Minuten oder weniger unter Verwendung eines Rollentrockners, um das Kollagenpulver zu erhalten.

2. Verfahren nach Anspruch 1, wobei die Trocknung unter Verwendung eines Rollentrockners mit einer Oberflächentemperatur von mehr als 150°C ausgeführt wird.

3. Verfahren nach dem vorhergehenden Anspruch, wobei die Temperatur der Schritte zur Herstellung des nassen Kollagenprodukts bei 45°C oder weniger und bevorzugt 40°C oder weniger gehalten wird.

4. Verfahren nach einem der vorhergehenden Ansprüche, wobei die Trocknung des nassen Kollagenprodukts bei einer Oberflächentemperatur des Rollentrockners von 155°C oder höher, bevorzugt 160°C oder höher, bevorzugter 165 ± 4°C ausgeführt wird.

5. Verfahren nach einem der vorhergehenden Ansprüche, wobei das Fell oder die Haut des Tieres ausgewählt ist aus der Gruppe bestehend aus Rind, Schwein und Geflügel, bevorzugt Rind.

6. Verfahren nach einem der vorhergehenden Ansprüche, wobei die Alkali- und/oder Oxidationsbehandlung bei einem pH-Wert von 10 oder höher, bevorzugt 11 oder höher, bevorzugter 12 oder höher durchgeführt wird.

7. Verfahren nach einem der vorhergehenden Ansprüche, wobei die Neutralisierungsbehandlung in einem pH-Wert von 6 oder weniger, bevorzugt von 5 bis 6 resultiert.

8. Verfahren nach einem der vorhergehenden Ansprüche, wobei das gemahlene Fell oder die Haut des Tiers innerhalb von 24 Stunden nach dem Mahlen, bevorzugt innerhalb von 12 Stunden, bevorzugter innerhalb von 6 Stunden und am meisten bevorzugt direkt nach dem Mahlen getrocknet wird.

9. Kollagenpulver, das ein Rind-, Schwein- oder Geflügelkollagenprodukt ist, erhältlich durch ein Verfahren nach einem der vorhergehenden Ansprüche, mit einer Gelfestigkeit nach Bloom von mindestens 300, wie bestimmt mittels Standard-Testverfahren ISO 9665 unter Verwendung eines Gels mit einer Konzentration von 6,67 %, das vor der Messung für 17 Stunden bei 10°C aufbewahrt wurde.

10. Kollagenpulver nach dem vorhergehenden Anspruch, das ein Rinderkollagen ist und das weniger als 1 Gew.-% Fett, Trockenmassebasis, und mindestens 95 Gew.-% Protein, Trockenmassebasis, umfasst; wie bestimmt durch das Nx5,52-Verfahren.

11. Kollagenpulver nach Anspruch 9 oder 10, wobei mehr als 99 Gew.-% Kollagen der Kollagenmoleküle dieselbe Molekularmasse haben.

12. Kollagenpulver nach dem vorhergehenden Anspruch, das ein Rinderkollagenpulverprodukt ist, wobei das Kollagen eine Molekularmasse von ungefähr 80 kDa hat.

13. Kollagenpulver nach einem der Ansprüche 9-12 zur Verwendung in der Verminderung von Hunger und zur Gewichtsregulierung, wobei bevorzugt das Nahrungsmittelprodukt zur Behandlung von Fettleibigkeit und metabolischem Syndrom bestimmt ist, insbesondere für die Behandlung von Insulinresistenz.

14. Nahrungsmittelprodukt mit einer stark sättigenden Wirkung, umfassend bis zu 5 Gew.-% Kollagenpulver nach einem der Ansprüche 9-12, bevorzugt 2-4 Gew.-%, bevorzugter 3 ± 0,5 Gew.-%, basierend auf dem Trockengewicht.

15. Nahrungsmittelprodukt nach Anspruch 14, wobei das Nahrungsmittelprodukt Zutaten umfasst, ausgewählt aus Gemüse, Obst, Eiern, Fleisch- oder Milchprodukten, Samen, Getreiden, Hülsenfrüchten, Fetten oder Ölen, Wasser, aromatischen Substanzen, Farb- und Aromastoffen, Lebensmittelsalzen, Ballaststoffen, Emulgatoren, Sacchariden, künstlichen und natürlichen Süßstoffen wie Zucker und Zuckerquellen, Kohlehydraten, Vitaminen, Mineralien und Kombinationen davon.

## Revendications

1. Procédé de préparation d'une poudre de collagène, comprenant les étapes suivantes :
- préparer, à une température inférieure ou égale à 50 °C, un produit collagène humide, en faisant subir à un cuir ou une peau une étape de réduction de taille, une étape de traitement alcalin et/ou oxydant, et une étape de neutralisation ;
- et faire ensuite sécher ce produit collagène humide, durant un laps de temps de 2 minutes ou moins, à l'aide d'un rouleau sécheur, de manière à obtenir ladite poudre de collagène.

2. Procédé conforme à la revendication 1, dans lequel ledit séchage est réalisé à l'aide d'un rouleau sécheur dont la température de surface est supérieure à 150 °C.

3. Procédé conforme à la revendication précédente, dans lequel la température, lors des étapes de préparation du produit collagène humide, est maintenue à 45 °C ou moins, et de préférence à 40 °C ou moins.

4. Procédé conforme à l'une des revendications précédentes, dans lequel ledit séchage dudit produit collagène humide est opéré à une température de surface dudit rouleau sécheur de 155 °C ou plus, de préférence de 160 °C ou plus, et mieux encore de 165 ± 4 °C.

5. Procédé conforme à l'une des revendications précédentes, dans lequel ledit cuir ou ladite peau d'animal est choisi(e) dans l'ensemble formé par ceux ou celles de bovins, de porcins et de volailles, et de préférence, est un cuir de bovin.

6. Procédé conforme à l'une des revendications précédentes, dans lequel ledit traitement alcalin et/ou oxydant est réalisé à un pH valant 10 ou plus, de préférence 11 ou plus, et mieux encore 12 ou plus.

7. Procédé conforme à l'une des revendications précédentes, dans lequel le traitement de neutralisation a pour résultat que le pH vaut 6 ou moins, et de préférence de 5 à 6.

8. Procédé conforme à l'une des revendications précédentes, dans lequel on fait sécher le cuir ou la peau d'animal broyé(e) dans les 24 heures suivant le broyage, de préférence dans les 12 heures, mieux encore dans les 6 heures, et surtout directement après le broyage.

9. Poudre de collagène qui est un produit collagène de bovin, de porcin ou de volaille, accessible par un procédé conforme à l'une des revendications précédentes, qui présente à l'état de gel une résistance à l'enfoncement (degré Bloom) d'au moins 300, valeur mesurée suivant le protocole d'essai de la norme ISO 9665 sur un gel dont la concentration vaut 6,67 % et qui a été maintenu à 10 °C durant 17 heures avant la mesure.

10. Poudre de collagène conforme à la revendication précédente, qui est un collagène de bovin et qui comprend moins de 1 % de matières grasses, en poids rapporté au poids à sec, et au moins 95 % de matières protéiques, en poids rapporté au poids à sec, ces teneurs étant déterminées suivant le procédé Nx5.52.

11. Poudre de collagène conforme à la revendication 9 ou 10, dans laquelle plus de 99 % en poids du collagène est constitué de molécules de collagène qui ont le même poids moléculaire.

12. Poudre de collagène conforme à la revendication précédente, qui est un produit collagène de bovin en poudre dans lequel le collagène présente une masse molaire d'environ 80 kDa.

13. Poudre de collagène conforme à l'une des revendications 9 à 12, pour utilisation en tant que coupe-faim et dans la maîtrise du poids, le produit alimentaire étant de préférence conçu pour le traitement d'une obésité ou d'un syndrome métabolique, en particulier pour le traitement d'une résistance à l'insuline.

14. Produit alimentaire à fort effet de satiété, qui comprend jusqu'à 5 %, de préférence de 2 à 4 % et mieux encore 3 ± 0,5 %, en poids rapporté au poids à sec, d'une poudre de collagène conforme à l'une des revendications 9 à 12.

15. Produit alimentaire conforme à la revendication 14, lequel produit alimentaire comprend des ingrédients choisis parmi les suivants : légumes, fruits, oeufs, viande ou produits laitiers, graines, grains, légumes secs, graisses ou huiles, eau, substances aromatiques, colorants et arômes, sels alimentaires, agents de charge, émulsifiants, saccharides, édulcorants artificiels ou naturels comme les sucres et les sources de sucres, glucides, vitamines et sels minéraux, ainsi que leurs combinaisons.
